# EUROPÄISCHE PATENTSCHRIFT

(11) **EP 2 225 219 B1**
(45) Veröffentlichungstag und Bekanntmachung des Hinweises auf die Patenterteilung: **16.03.2016**
(21) Anmeldenummer: 08861318.7
(22) Anmeldetag: 17.12.2008
(51) Int. Cl.: C07D 309/04

(54) **VERFAHREN ZUR HERSTELLUNG VON CIS-ROSENOXID**
PROCESS FOR THE PREPATAION OF CIS-ROSE OXIDE
PROCEDE DE PREPARATION D'OXYDE DE ROSE A CONFIGURATION CIS

(30) Priorität: 19.12.2007 EP 07123639
(43) Veröffentlichungstag der Anmeldung: 08.09.2010
(73) Patentinhaber: BASF SE, 67056 Ludwigshafen (DE)
(72) Erfinder: KÖNIGSMANN, Lucia, 68159 Mannheim (DE); SCHUBERT, Jürgen, 67246 Dirmstein (DE); WALCH, Andreas, 74193 Schwaigern (DE); GOTTWALD, Günther, 68167 Mannheim (DE); KAMASZ, Martin, 68307 Mannheim (DE); SCHWAB, Ekkehard, 67434 Neustadt (DE); PFAFF, Klaus-Peter, 67159 Friedelsheim (DE); SLANY, Michael, 67281 Kirchheim (DE)
(86) Internationale Anmeldenummer: PCT/EP2008/067688
(87) Internationale Veröffentlichungsnummer: WO 2009/077550

(56) Entgegenhaltungen:
- WO-A1-97/00509

## Beschreibung

Die vorliegende Erfindung betrifft ein Verfahren zur Herstellung von cis-2-(2-Methyl-prop-1-en-yl)-4-methyl-tetrahydropyran umfassend die katalytische Hydrierung von 2-(2-Methyl-prop-1-en-yl)-4-methylen-tetrahydropyran in Gegenwart von Wasserstoff und eines heterogenen, Ruthenium auf einem Träger umfassenden Katalysators und anschließendes Inkontaktbringen der so erhaltenen Verbindungen mit einem stark sauren Kationenaustauscher.

Cis-2-(2-Methyl-prop-1-en-yl)-4-methyl-tetrahydropyran stellt eine wertvolle, auch als Rosenoxid bezeichne Aromachemikalie dar. Sie fällt üblicherweise in Form eines Diastereomerengemisches mit der entsprechenden trans-konfigurierten Verbindung an, wobei sich die genannte cis-konfigurierte Verbindung als die wertvollere, weil besser duftende Verbindung erwiesen hat. Da sich die genannten Diastereomere insbesondere bei Herstellung im technischen Maßstab nur schwer voneinander trennen lassen besteht nachhaltiger Bedarf an Herstellverfahren, bei denen in hoher Ausbeute möglichst selektiv das bevorzugte cis-Isomere (Rosenoxid) gebildet wird.

J.H.P. Tyman and B.J. Williis beschrieben in Tetrahedron Letters No. 51, 4507 - 4508, 1970 die Säure-katalysierte Umsetzung von 3-Alken-1-olen mit Aldehyden, speziell die Umsetzung von 3-Methyl-2-buten-1-al mit 2-Methyl-1-buten-4-ol und anschließender Dehydratisierung. Das so erhaltene, eine exocyclische Methylengruppe aufweisende Zwischenprodukt wurde homogenkatalytisch in Gegenwart von SnCl₂/H₂PTCl₆ zum racemischen cis-2-(2'-methyl-1'-propenyl)-4-methyltetrahydropyran hydriert.

Die WO 79/00509 offenbart ein Verfahren zur Herstellung von bezüglich des cis-Isomeren angereicherten Gemischen von cis- und trans-(2-Methyl-prop-1-en-yl)-4-methyl-tetrahydropyran durch katalytische Hydrierung der entsprechenden, in 4-position eine exo-Methylengruppe aufweisenden Vorstufe. Als geeignete Hydrierkatalysatoren werden Raney-Nickel und Palladiumkatalysatoren, speziell Palladium auf Kohlenstoff, genannt. Die Isomerenanreicherung wird durch Behandlung des Hydrierproduktes mit einem sauren oder Lewis-sauren Reagenz erreicht. Als bevorzugte Lewis-Säure wird Bortrifluorid genannt.

Die beispielhaft beschriebene Hydrierung mit Raney-Nickel mit anschließender Destillation liefert in einer Ausbeute von 87,9 % d.Th. ein Gemisch der cis- und trans-Isomere in einem Verhältnis von 4 : 6. Dieses Gemisch wird in der anschließenden Isomerisierung in einer Ausbeute von 86,5% d.Th. in ein Isomerengemisch im Verhältnis von etwa 85 : 15 umgesetzt.

Die EP 0 082 401 A1 offenbart ein Verfahren zur Herstellung von überwiegend, d.h. zu mindestens 85% das Z-Isomere enthaltendem Rosenoxid. Das Verfahren ist dadurch gekennzeichnet, dass man 2-[2-Methyl-prop-1-en-yl]-4-methylen-tetrahydropyran ("Dehydrorosenoxid") an einem Platindioxid- oder einem Platin/Kohle-Katalysator in Gegenwart eines stark sauren Kationenaustauschers hydriert.

Bei der beispielhaft beschriebenen isomerisierenden Hydrierung von Dehydrorosenoxid zu Rosenoxid wird eine Ausbeute von bis zu 87% d.Th. mit einem Isomerengehalt des E-isomeren von 90,5 % bzw. des Z-Isomeren von 7% erreicht.

Ausgehend von diesem Stand der Technik bestand die Aufgabe der vorliegenden Erfindung darin, ein Verfahren zur Herstellung von diastereomerenangereichertem Rosenoxid bereitzustellen, das sich auf verfahrenstechnisch gut handhabbare Weise und mit hoher Gesamtausbeute bei möglichst hoher Selektivität bezüglich des gewünschten cis-2-(2-Methyl-prop-1-en-yl)-4-methyl-tetrahydropyrans im technischen Maßstab durchführen lässt. Dabei sollen wohlfeile, leicht wiederzugewinnende und gut wieder einsetzbare Ausgangsverbindungen und Reagenzien bzw. Katalysatoren genutzt werden können.

Die Aufgabe wurde erfindungsgemäß gelöst durch die Bereitstellung eines Verfahrens zur Herstellung von cis-2-(2-Methyl-prop-1-en-yl)-4-methyl-tetrahydropyran der Formel (I) 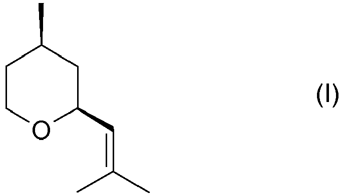 umfassend die Schritte
a) katalytische Hydrierung von 2-(2-Methyl-prop-1-en-yl)-4-methylen-tetrahydropyran der Formel (II) 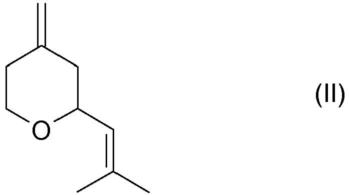 in Gegenwart von Wasserstoff und eines heterogenen, Ruthenium auf einem Träger umfassenden Katalysators unter Erhalt eines Reaktionsgemisches, umfassend die Verbindung der Formel (I) und trans-2-(2-Methyl-prop-1-en-yl)-4-methyl-tetrahydropyran der Formel (III) 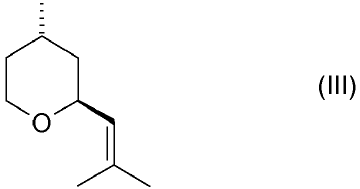 und
b) gegebenenfalls Abtrennung der Verbindungen der Formel (I) und (III) von dem gemäß Schritt a) erhaltenen Reaktionsgemisch und
c) Inkontaktbringen der in Schritt a) oder b) erhaltenen Verbindungen der Formeln (I) und (III) mit einem stark sauren Kationenaustauscher unter Isomerisierung der Verbindung der Formel (III) zur Verbindung der Formel (I).

Das erfindungsgemäße Verfahren eignet sich zur Herstellung von cis-2-(2-Methyl-prop-1-en-yl)-4-methyl-tetrahydropyran der Formel (I) 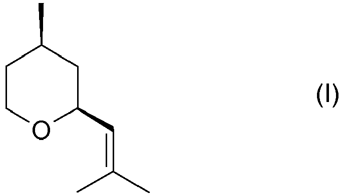 welches im Folgenden auch als Rosenoxid bezeichnet wird und das üblicherweise in Form von Gemischen mit seinem Diastereomeren trans-2-(2-Methyl-prop-1-en-yl)-4-methyl-tetrahydropyran der Formel (III) 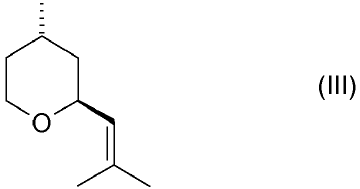 anfällt.

Im Rahmen einer bevorzugten Ausführungsform betrifft das erfindungsgemäße Verfahren ein Verfahren zur Herstellung eines Isomerengemisches von cis-2-(2-Methyl-prop-1-en-yl)-4-methyl-tetrahydropyran der Formel (I) und trans-2-(2'-Methyl-prop-1-en-yl)-4-methyl-tetrahydropyran der Formel (III). Im Rahmen einer besonders bevorzugten Ausführungsform betrifft das erfindungsgemäße Verfahren ein Verfahren zur Herstellung eines Isomerengemisches von cis-2-(2-Methyl-prop-1-en-yl)-4-methyl-tetrahydropyran der Formel (I) und trans-2-(2-Methyl-prop-1-en-yl)-4-methyl-tetrahydropyran der Formel (III), enthaltend, bezogen auf die Menge des Isomerengemisches, mindestens 70%, bevorzugt mindestens 90% und besonders bevorzugt 90 bis 98% cis-2-(2-Methyl-prop-1'-en-yl)-4-methyl-tetrahydropyran der Formel (I) und höchstens 30%, bevorzugt höchstens 10% und besonders bevorzugt 2 bis 10% trans-2-(2-Methyl-prop-1-en-yl)-4-methyl-tetrahydropyran der Formel (III).

Das genannte Diastereomerengemisch fällt dabei im Rahmen des erfindungsgemäßen Herstellverfahrens üblicherweise in hoher chemischer Reinheit von in der Regel 90 Gew.-% oder darüber, bevorzugt 95 bis 99,9 Gew.-% und besonders bevorzugt 97 bis 99,5 Gew.-% (jeweils bezogen auf die Summe der beiden Diastereomere) an.

Die Verbindungen der Formeln (I) und (III) fallen erfindungsgemäß in racemischer Form an. Die Formelbilder (I) und (III) dienen dementsprechend der Veranschaulichung der relativen Konfiguration der beiden Stereozentren und stehen jeweils für die racemischen Gemische der jeweiligen Enantiomerenpaare. Als erfindungsgemäß einzusetzender Ausgangsstoff dient daher auch racemisches 2-(2-Methyl-prop-1-en-yl)-4-methylen-tetrahydropyran der Formel (II), das im Prinzip auf jedwede Weise hergestellt sein kann und an dessen Beschaffenheit oder Reinheit im Rahmen des für synthetische Zwecke Üblichen keine speziellen Anforderungen zu stellen sind.

Im Rahmen einer bevorzugten Ausführungsform umfasst das erfindungsgemäße Verfahren als zusätzlichen, vorgeschalteten Verfahrensschritt auch die Bereitstellung von 2-(2-Methyl-prop-1-en-yl)-4-methylen-tetrahydropyran (Dehydrorosenoxid) der Formel (II) durch Umsetzung von 3-Methyl-but-3-en-1-ol (Isoprenol) der Formel (IV) 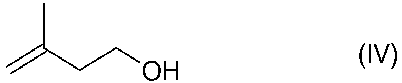 mit 3-Metyhl-but-2-en-1-al (Prenal) der Formel (V) 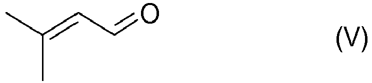 unter Freisetzung von Wasser in Gegenwart einer Säure und in Gegenwart eines mit Wasser ein Azeotrop bildenden Lösungsmittels.

Im Rahmen einer besonders bevorzugten Ausführungsform des erfindungsgemäßen Verfahrens geht man dabei so vor, dass man das bei der vorstehend genannten Umsetzung von 3-Methyl-but-3-en-1-ol (Isoprenol) der Formel (IV) mit 3-Metyhl-but-2-en-1-al (Prenal) der Formel (V) freigesetzte Wasser durch Azeotropdestillation mit dem eingesetzten Lösungsmittel vom Reaktionsgemisch abtrennt. Dabei können sowohl einzelne, mit Wasser ein Azeotrop bildende Lösungsmittel als auch Gemische verschiedener Lösungsmittel eingesetzt werden. Bevorzugt setzt man dazu solche Lösungsmittel ein, die mit Wasser ein Azeotrop bilden, das einen niedrigeren Siedepunkt als das jeweilige Lösungsmittel bzw. Lösungsmittelgemisch selbst aufweist, vorzugsweise solche, deren azeotroper Siedepunkt im Bereich von etwa 60°C bis etwa 120°C, besonders bevorzugt im Bereich von etwa 65°C bis etwa 90°C liegt. Als im Rahmen dieser Ausführungsform bevorzugt einsetzbare Lösungsmittel seien beispielsweise solche genannt, die ausgewählt sind aus der Gruppe der Lösungsmittel umfassend Ethanol, Benzol, Tetrachlorkohlenstoff, Essigsäureethylester,Toluol, Chloroform, n-Heptan, Cyclohexan und Methylcyclohexan. Als besonders bevorzugte, mit Wasser ein Azeotrop bildende Lösungsmittel seien solche genannte, die ausgewählt sind aus der Gruppe umfassend Toluol, Chloroform, n-Heptan, Cyclohexan und Methylcyclohexan. Als ganz besonders bevorzugte Lösungsmittel seien Toluol und n-Heptan, insbesondere bevorzugt Toluol genannt.

Die Abtrennung des bei der Reaktion freigesetzten Wassers durch Azeotropdestillation kann nach dem Fachmann an sich bekannten Verfahren bzw. mit den für diesen Zweck geeigneten Vorrichtungen wie beispielsweise mit einem Wasserabscheider durchgeführt werden.

Die im Rahmen der vorstehend genannten Azeotropdestillation einzusetzende Menge an jeweils gewähltem Lösungsmittel bzw. Lösungsmittelgemisch kann in einem breiten Bereich gewählt werden und richtet sich in der Regel nach den gewählten Reaktionsbedingungen sowie der zur Wasserabtrennung eingesetzten Vorrichtung. Es hat sich als vorteilhaft erwiesen, das gewählte Lösungsmittel bzw. Lösungsmittelgemisch in einer auf die Gesamtmenge der eingesetzten Edukte 3-Methyl-but-3-en-1-ol (Isoprenol) und 3-Metyhl-but-2-en-1-al (Prenal) bezogenem Mengenverhältnis von etwa 1 zu 1 bis etwa 2 zu 1, bevorzugt etwa 1 zu 1 bis etwa 1,5 zu 1, d.h. in leichtem Überschuss, einzusetzen. Das Lösungsmittel kann nach Durchführung der Reaktion in der Regel gut abgetrennt und im Rahmen weiterer Umsetzungen wieder eingesetzt werden.

Die optional durchzuführende Bereitstellung von Dehydrorosenoxid der Formel (II) durch Umsetzung von 3-Methyl-but-3-en-1-ol (Isoprenol) und 3-Metyhl-but-2-en-1-al (Prenal) wird auch in Gegenwart einer Säure durchgeführt. Als geeignete Säuren haben sich hierzu sowohl organische als auch anorganische Säuren wie beispielsweise p-Toluolsulfonsäure, Triflouressigsäure oder auch Alkalihydrogensulfate erwiesen. Im Rahmen einer bevorzugten Ausführungsform führt man die Umsetzung von 3-Methyl-but-3-en-1-ol mit 3-Methyl-but-2-en-1-al in Gegenwart eines Alkalihydrogensulfates wie beispielsweise Natriumhydrogensulfat oder Kaliumhydrogensulfat, bevorzugt Natriumhydrogensulfat durch.

Die gewählte Säure wird bevorzugt in katalytischen Mengen eingesetzt, üblicherweise, bezogen auf die Gesamtmenge der umzusetzenden Ausgangsstoffe 3-Methyl-but-3-en-1-ol und 3-Metyhl-but-2-en-1-al, in einer Menge von etwa 0,01 bis etwa 1 Gew.-%.

Die Reaktion zur Herstellung von Dehydrorosenoxid durch Kondensation von Isoprenol mit Prenal wird in der Regel und in Abhängigkeit vom gewählten Lösungsmittel bzw. Lösungsmittelgemisch und der gewählten Säure bei Temperaturen im Bereich von etwa 60°C bis 150°C, bevorzugt im Bereich von etwa 70°C bis 120°C durchgeführt und ist dann in der Regel rasch, oft nach etwa 24 h oder auch früher weitgehend abgeschlossen. Das erhaltene Reaktionsgemisch kann nach dem Fachmann bekannten Verfahren, beispielsweise durch extraktive Verfahren, gegebenenfalls nach Neutralisation der eingesetzten Säure aufgearbeitet werden. Das so als Rohprodukt erhaltene Dehydrorosenoxid der Formel (II) kann im Anschluss weiter aufgereinigt werden, beispielsweise durch Chromatographie oder bevorzugt durch (fraktionierte) Destillation, wobei insbesondere das als Nebenprodukt üblicherweise anfallende Neroloxid sowie weitere hochsiedende Nebenkomponenten abgetrennt werden können.

Die gemäß Verfahrensschritt a) des erfindungsgemäßen Verfahrens durchzuführende katalytische Hydrierung von 2-(2-Methyl-prop-1-en-yl)-4-methylen-tetrahydropyran der Formel (II) unter Erhalt eines Reaktionsgemisches, umfassend die Verbindung der Formel (I) und die Verbindung der Formel (III) wird in Gegenwart von Wasserstoff und in Gegenwart eines heterogenen, Ruthenium auf einem Träger umfassenden Katalysators durchgeführt. Bevorzugt setzt man dabei solche Katalysatoren ein, die Ruthenium auf einem Kohlenstoffträger umfassen. Die genannten Katalysatoren können gegebenenfalls noch weitere Metalle, beispielsweise in Form von Dotierungen umfassen. Unter dem Begriff Träger sind dabei die dem Fachmann geläufigen Trägermaterialen wie beispielsweise SiO₂, Al₂O₃, Graphite, Ruße oder Aktivkohle genannt. Als bevorzugte Träger sind darunter Kohlenstoffträger, d.h. die dem Fachmann geläufigen auf Kohlenstoff basierenden Trägermaterialen zu verstehen wie beispielsweise Aktivkohle, Graphite oder Ruße. Als bevorzugter Kohlenstoffträger sei Aktivkohle wie beispielsweise Norit® SX Plus genannt.

Im Rahmen einer bevorzugten Ausführungsform führt man Verfahrensschritt a) in Gegenwart eines mit Eisen dotierten Ruthenium auf einem Träger umfassenden Katalysators durch. Besonders bevorzugt führt man den erfindungsgemäßen Verfahrensschritt a) in Gegenwart eines mit Eisen dotierten Ruthenium auf einem Kohlenstoffträger umfassenden Katalysators durch. Derartige Katalysatoren sind bekannt und beispielsweise in der EP 0 071 787 bzw. der EP 1 317 959, auf die hiermit diesbezüglich vollumfänglich Bezug genommen wird, beschrieben. Erfindungsgemäß besonders bevorzugt einzusetzende Katalysatoren sind solche, die, jeweils bezogen auf den fertigen Katalysator, 0,1 bis 10 Gew.-% Ruthenium und 0,1 bis 5 Gew.-% Eisen, insbesondere bevorzugt 4 bis 6 Gew.-% Ruthenium und 0,5 bis 1,5 Gew.-% Eisen auf einem Kohlenstoffträger, bevorzugt auf Aktivkohle enthalten.

Die erfindungsgemäß gemäß Verfahrenschritt a) durchzuführende katalytische Hydrierung wird üblicherweise bei Temperaturen im Bereich von etwa 50°C bis etwa 150 °C, bevorzugt im Bereich von etwa 70°C bis etwa 130°C und bei absoluten Drucken im Bereich von etwa 1 bis etwa 25 bar, bevorzugt im Bereich von etwa 2 bis etwa 10 bar durchgeführt. Die erfindungsgemäß durchzuführende katalytische Hydrierung kann auch in Gegenwart von unter den Reaktionsbedingungen inerten Lösungsmitteln wie beispielsweise Methanol, Hexan, Tetrahydrofuran durchgeführt werden.

Der einzusetzende Wasserstoff kann in reiner Form oder gewünschtenfalls auch in Form von Gemischen mit anderen, bevorzugt inerten Gasen wie Stickstoff oder Argon, eingesetzt werden. Bevorzugt setzt man Wasserstoff in unverdünnter Form ein.

Nach Abtrennung des eingesetzten Katalysators, beispielsweise durch Filtration und gegebenenfalls Abtrennung des eingesetzten Lösungsmittels, vorzugsweise durch Destillation, erhält man ein Reaktionsgemisch, das die diastereomeren Verbindungen der Formeln (I) und (III) enthält und gegebenenfalls noch weitere Verunreinigungen, unerwünschte Nebenkomponenten oder auch Reste nicht umgesetzten Eduktes enthalten kann.

Gemäß dem optional durchzuführenden Verfahrensschritt b) des erfindungsgemäßen Verfahrens kann man gewünschtenfalls eine Abtrennung der Verbindungen der Formel (I) und (III) von dem gemäß Schritt a) erhaltenen Reaktionsgemisch durchführen. Dazu stehen die dem Fachmann als geeignet erscheinenden Methoden der Stofftrennung zur Verfügung wie beispielsweise Chromatographie oder bevorzugt Destillation zur Verfügung. Als geeignete Destillationsvorrichtungen seien beispielsweise Vorrichtungen zur Kurzwegdestillation wie beispielsweise Dünnschichtverdampfer genannt oder auch gefüllte oder gepackte Kolonnen sowie Bodenkolonnen.

Das auf diese Weise gemäß Verfahrenschritt a) oder nach Aufreinigung gemäß dem optionalen Verfahrensschritt b) erhaltenen Gemisch der Verbindungen der Formel (I) und (III) wird anschließend gemäß dem separaten Verfahrensschritt c) mit einem stark sauren Kationenaustauscher unter Isomerisierung der Verbindung der Formel (III) zur Verbindung der Formel (I) in Kontakt gebracht.

Dabei kann die Verbindung der Formel (III), d.h. das trans-Diastereomer ganz oder teilweise, in der Regel teilweise, in ihr Diastereomer der Formel (I), d.h. in das cis-Diastereomer, überführt werden. Man erhält dadurch nach Durchführung des separaten Verfahrensschritts c) des erfindungsgemäßen Verfahrens Gemische der Verbindungen der Formeln (I) und (III), die einen höheren Gehalt der gewünschten Verbindung der Formel (I) aufweisen als die zunächst durch die Verfahrensschritt a) bzw. b) erhaltenen Gemische. Man erhält auf diese Weise bevorzugt die vorstehend genannten diastereomerenangereicherten Gemische von cis-2-(2-Methyl-prop-1-en-yl)-4-methyl-tetrahydropyran der Formel (I) und trans-2-(2-Methyl-prop-1-en-yl)-4-methyl-tetrahydropyran der Formel (III), enthaltend, bezogen auf die Menge des Isomerengemisches, mindestens 70%, bevorzugt mindestens 90% und besonders bevorzugt 90 bis 98% cis-2-(2-Methyl-prop-1-en-yl)-4-methyl-tetrahydropyran der Formel (I) und höchstens 30%, bevorzugt höchstens 10% und besonders bevorzugt 2 bis 10% trans-2-(2-Methyl-prop-1-en-yl)-4-methyl-tetrahydropyran der Formel (III).

Die Isomerisierung gemäß Verfahrensschritt c) des erfindungsgemäßen Verfahrens erfolgt in Gegenwart eines stark sauren Ionentauschers, d.h. eines stark sauren Kationentauschers wie beispielsweise Lewatit® S100, Lewatit® SP112, Lewatit® S115, Lewatit® SP1080, Lewatit® SC102, Lewatit® SPC118, Lewatit® CNP 80, Lewatit® HD 5, Amberlite® IR 120, Amberlite® IR200, Amberlyst™ 15, Bay. KAT. K 2431, Bay. KAT. K 2621, Dowex® 50, Permutit® RS, Wofatit® KPS 200, Duolite™ C-3, Duolite™ C-10, Duolite™ C-25, Wofatit® F, Wofatit® D, Wofatit® P, Zeoxex (Zeo karb H), Nalcite HCR, Nalcite HGR, Nalcite HDR, Permutit® Q und Permutit® RS, Serdrolit® rot. Die gewählten stark sauren Kationentauscher können auch in Form von Gemischen zweier oder mehrerer verschiedener Kationentauscher eingesetzt werden. Erfindungsgemäß bevorzugt setzt man die Kationenaustauscher Lewatit® SP112 und/oder Amberlyst™ 15 ein.

Im Rahmen einer bevorzugten Ausführungsform des erfindungsgemäßen Verfahrens setzt man den gewählten Kationentauscher in Form eines Festbetts ein, über das das umzusetzende, aus Verfahrensschritt a) bzw. b) erhaltene Diastereomerengemisch als solches oder in Form einer Lösung in einem geeigneten, unter den Reaktionsbedingungen inerten Lösungsmittel, geleitet wird. Bevorzugt bringt man das zu isomerisierende Gemisch in unverdünnter Form mit dem gewählten, stark sauren Kationentauscher in Kontakt. Dabei kann das Festbett beispielsweise in Form einer Schüttung des gewählten Kationentauschers in einem Reaktionsrohr ausgestaltet sein, wobei das zu isomerisierende Gemisch durch das so befüllte Reaktionsrohr geleitet wird. Dazu können die Reaktoren in allen dem Fachmann geeignet erscheinenden Betriebs- bzw. Fahrweisen betrieben werden wie beispielsweise in Sumpffahrweise oder erfindungsgemäß bevorzugt in Rieselfahrweise, wobei das zu isomerisierende Gemisch auf eine Schüttung des gewählten Kationentauschers gerieselt wird.

Auf diese Weise ist auch die erfindungsgemäß im Rahmen von Verfahrensschritt c) bevorzugte kontinuierliche Reaktionsführung möglich. Im Rahmen einer bevorzugten Ausführungsform führt man daher Verfahrensschritt c) kontinuierlich durch. Dabei wird das zu isomerisierende, die Verbindungen der Formel (I) und (III) enthaltende Gemisch kontinuierlich dem Kationentauscher zugeführt, beispielsweise durch Eintragen in einen mit Kationentauscher befüllten Reaktor und kontinuierlich von demselben wieder abgetrennt, beispielsweise durch Austragen des isomerisierten Gemisches aus dem Reaktor.

Das zu isomerisierende Gemisch der Verbindungen der Formeln (I) und (III) kann auch mehrmals hintereinander mit dem gewählten stark sauren Kationentauscher oder auch mit verschiedenen stark sauren Kationentauschern in Kontakt gebracht werden, beispielsweise durch Rückführung des aus dem wie vorstehend beschriebenen Festbettreaktor ausgetragenen, diastereomerenangereicherten Isomerengemisch in den gleichen Reaktor. Es ist auch möglich, mehrere derartige Reaktoren, die gewünschtenfalls auch mit verschiedenen Kationentauschern befüllt sein können, hintereinander zu durchlaufen, um so zu dem gewünschten, wie vorstehend beschriebenen Diastereomerenverhältnis zu gelangen.

Die Isomerisierung gemäß Verfahrensschritt c) wird üblicherweise bei Temperaturen von etwa 0°C bis etwa 100°C, bevorzugt bei etwa 20 bis etwa 80 °C durchgeführt.

Die folgenden Beispiele dienen der Verdeutlichung der Erfindung, ohne sie in irgendeiner Weise zu beschränken:

### Beispiel 1:

In einem mit Rührer, Wasserauskreiser, Kühler und einer Dosierpumpe versehenen Reaktionsgefäß mit einem Volumen von 5 I wurden 2000 g Toluol und 1,5 g NaHSO₄ (als 10%-ige wässrige Lösung) vorgelegt und 7,67 mol (660 g) 3-Methyl-but-3-en-1-ol und 7,67 mol (643,5 g) 3-Methyl-but-2-en-1-al innerhalb 16 h bei 110 bis 115°C zudosiert. Das Wasser wurde kontinuierlich aus dem Reaktionsgemisch mit Toluol ausgekreist und das Toluol zurückgeführt. In Anschluss wurde das Reaktionsgemisch weitere 5,5 h bei 115°C gerührt. Das erhaltene Reaktionsgemisch wurde anschließend mit 278 g 2%-iger NaOH-Lösung gewaschen. Das Toluol wurde bei einem Druck von 200 mbar über einer 30 cm lange, mit Raschigringen gefüllte Kolonne, abdestilliert. Der Umsatz zur Dehydrorosenoxid (DHR) betrug 62,7 % d.Th. Abschließend wurde das DHR durch Destillation von Neroloxid und hochsiedenden Nebenkomponenten getrennt und mit einer Reinheit von >99% erhalten.

### Beispiel 2:

Das so erhaltene Dehydrorosenoxid (DHR) wurde in einem 500 ml Büchi-Laborautoklaven bei einem Wasserstoffdruck von 4 bar (Versuch a)) bzw. 3 bar (Versuche b) und d)) und einer Temperatur von 100°C, unter kräftigem Rühren durchgeführt. Nach Beendigung der Reaktion wurde der Katalysator abfiltriert.

### Beispiel 2a:

74,2 g Dehydrorosenoxid gelöst in 112 g Methanol wurden wie vorstehend beschrieben in Gegenwart von 1,1 g eines Katalysators von 5 Gew.-% Ruthenium und 1 Gew.-% Eisen auf Aktivkohle hydriert. Das erhaltenen Reaktionsgemisch wurde zu den in Tabelle 1 angegeben Zeiten gaschromatographisch analysiert (GC-Metode: Säule: DB-210, 30 m, 0,32 mm, 0,5 µm; 50°C, mit 3°C/min auf 230°C). Man erhielt die in Tabelle 1 angegebenen Ergebnisse (jeweils in GC-Flächen-%, Umsatz und Selektivität jeweils in %):

**Tabelle 1:**

| Zeit (min) | 30 | 60 | 180 | 240 | 300 | 360 |
|---|---|---|---|---|---|---|
| Dehydrorosenoxid | 75,57 | 73,63 | 48,53 | 12,85 | 6,74 | 0,00 |
| cis-Rosenoxid | 7,32 | 8,06 | 16,74 | 28,94 | 31,08 | 32,32 |
| trans-Rosenoxid | 13,98 | 15,28 | 30,68 | 52,44 | 55,99 | 57,79 |
| Umsatz | 24,23 | 26,37 | 51,47 | 87,15 | 93,26 | 100 |
| Selektivität | 87,91 | 88,51 | 92,13 | 93,38 | 93,36 | 90,11 |

### Beispiel 2 b)

270 g Dehydrorosenoxid wurden ohne Zusatz eines Lösungsmittels in Gegenwart von 1 Gew.-% des unter Beispiel 2a) beschriebenen Katalysators hydriert. Man erhielt die in Tabelle 2 angegeben Ergebnisse:

**Tabelle 2:**

| Zeit (min) | 30 | 60 | 90 | 110 |
|---|---|---|---|---|
| Dehydrorosenoxid | 59,28 | 20,03 | 5,10 | 0,33 |
| cis-Rosenoxid | 10,48 | 20,64 | 23,92 | 24,55 |
| trans-Rosenoxid | 25,07 | 50,38 | 58,19 | 59,08 |
| Umsatz | 40,72 | 79,97 | 94,90 | 99,67 |
| Selektivität | 87,30 | 88,80 | 86,53 | 83,90 |

### Beispiel 2c)

Der im vorstehend beschriebenen Beispiel 2b) eingesetzte und durch Abfiltrieren abgetrennte Katalysator wurde in diesem Versuch unter ansonsten unveränderten Bedingungen wieder eingesetzt. Man erhielt die in Tabelle 3 angegebenen Ergebnisse:

**Tabelle 3:**

| Zeit (min) | 30 | 60 | 90 | 110 |
|---|---|---|---|---|
| Dehydrorosenoxid | 61,65 | 21,45 | 1,77 | 0,11 |
| cis-Rosenoxid | 9,82 | 20,46 | 25,06 | 24,88 |
| trans-Rosenoxid | 23,88 | 50,35 | 61,50 | 60,51 |
| Umsatz | 38,35 | 78,55 | 98,23 | 99,89 |
| Selektivität | 87,87 | 90,15 | 88,12 | 85,48 |

### Beispiel 3: Isomerisierung von cis/trans Rosenoxid

In einem 1l Kolben wurden 452,4 g eine Gemisches von cis- und trans-Rosenoxid (Verhältnis 0,4 zu 1) mit 4,5 g Ionenaustauscher SP112 H-Form bei 50°C über eine Gesamtversuchsdauer von 6 h gerührt. Der Ionenaustauscher Lewatit®SP112 H-Form wurde vor dem Einsatz mehrfach mit Methanol wasserfrei gewaschen. Die gaschromatographisch ermittelten Ergebnisse sind in Tabelle 4 dargestellt:

**Tabelle 4:**

| Versuchsdauer [h] | Umsatz trans-Rosenoxid [%] | Selektivität zu cis-Rosenoxid [%] | Verhältnis cis/trans [x zu 1] |
|---|---|---|---|
| 0 | 0,0 | 0,0 | 0,4 |
| 1 | 56,5 | 81,5 | 2,0 |
| 1,7 | 69,3 | 78,2 | 3,1 |
| 3,25 | 80,7 | 72,6 | 5,2 |
| 4 | 85,2 | 69,4 | 6,8 |
| 5 | 88,7 | 65,0 | 8,8 |
| 6 | 90,3 | 62,0 | 10,0 |

### Beispiel 4: Isomerisierung von cis/trans Rosenoxid mittels Festbett-Ionentauscher

Als Reaktor diente ein mit 3 g Ionenaustauscher Lewatit® SP112 H-Form befülltes, beheiztes Reaktionsrohr aus Edelstahl (Länge 200 mm; Innendurchmesser 6 mm), das mit einer Zulaufpumpe für das Rosenoxid (30 g) und mit einem Vorlagebehälter mit Probennahme ausgerüstet war. Der Reaktor wurde gerieselt betrieben. Das Rosenoxid wurde bei 55°C im Kreis über das Festbett gefahren bis ein Verhältnis von cis- zu trans-Rosenoxid von >10 zu 1 erreicht wurde. Man erhielt die in Tabelle 5 zusammengestellten, gaschromatographisch ermittelten Ergebnisse:

**Tabelle 5:**

| Versuchsdauer [h] | Umsatz trans-Rosenoxid [%] | Selektivität zu cis-Rosenoxid [%] | Verhältnis cis/trans [x zu 1] |
|---|---|---|---|
| 0 | 0,0 | 0,0 | 2,3 |
| 1 | 44,6 | 73,8 | 4,7 |
| 3 | 67,0 | 79,7 | 8,5 |
| 5 | 75,4 | 78,1 | 11,6 |

Nach 5 Stunden wurde ein Verhältnis von cis- zu trans-Rosenoxid von 11,6 zu 1 erreicht. Abschließend wurde das erhaltene Rosenoxid durch Destillation von Nebenkomponenten getrennt. Man erhielt eine Ausbeute nach Destillation von 92,12 % cis-Rosenoxid und 91,67 % trans-Rosenoxid.

## Patentansprüche

1. Verfahren zur Herstellung von cis-2-(2-Methyl-prop-1-en-yl)-4-methyl-tetrahydropyran der Formel (I) 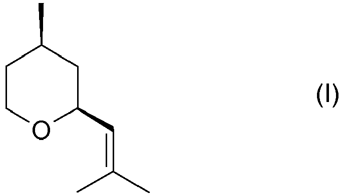 umfassend die Schritte
a) katalytische Hydrierung von 2-(2-Methyl-prop-1-en-yl)-4-methylen-tetrahydropyran der Formel (II) 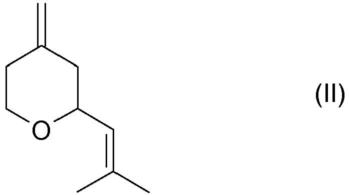 in Gegenwart von Wasserstoff und eines heterogenen, Ruthenium auf einem Träger umfassenden Katalysators unter Erhalt eines Reaktionsgemisches, umfassend die Verbindung der Formel (I) und trans-2-(2-Methyl-prop-1-en-yl)-4-methyl-tetrahydropyran der Formel (III) 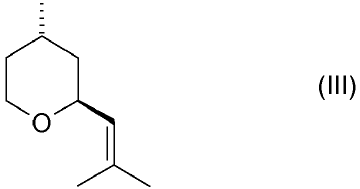 und
b) gegebenenfalls Abtrennung der Verbindungen der Formel (I) und (III) von dem gemäß Schritt a) erhaltenen Reaktionsgemisch und
c) Inkontaktbringen der in Schritt a) oder b) erhaltenen Verbindungen der Formeln (I) und (III) mit einem stark sauren Kationenaustauscher unter Isomerisierung der Verbindung der Formel (III) zur Verbindung der Formel (I).

2. Verfahren nach Anspruch 1, zusätzlich umfassend die Bereitstellung der Verbindung der Formel (II) durch Umsetzung von 3-Methyl-but-3-en-1-ol mit 3-Methyl-but-2-en-1-al unter Freisetzung von Wasser in Gegenwart einer Säure und eines mit Wasser ein Azeotrop bildenden Lösungsmittels.

3. Verfahren nach Anspruch 2, **dadurch gekennzeichnet, dass** man das bei Umsetzung von 3- Methyl-but-3-en-1-ol mit 3-Methyl-but-2-en-1-al freigesetzte Wasser durch Azeotropdestillation mit dem eingesetzten Lösungsmittel vom Reaktionsgemisch abtrennt.

4. Verfahren nach Anspruch 2 oder 3, **dadurch gekennzeichnet, dass** man ein mit Wasser ein Azeotrop bildenden Lösungsmittel einsetzt, ausgewählt aus der Gruppe der Lösungsmittel umfassend Toluol, Chloroform, n-Heptan, Cyclohexan, und Methylcyclohexan.

5. Verfahren nach einem der Ansprüche 2 bis 4, **dadurch gekennzeichnet, dass** man die Umsetzung von 3-Methyl-but-3-en-1-ol mit 3-Methyl-but-2-en-1-al in Gegenwart von p-Toluolsulfonsäure, Triflouressigsäure oder einem Alkalihydrogensulfat durchführt.

6. Verfahren nach einem der Ansprüche 2 bis 5, **dadurch gekennzeichnet, dass** man ein Alkalihydrogensulfat als Säure einsetzt.

7. Verfahren nach einem der Ansprüche 2 bis 6, **dadurch gekennzeichnet, dass** man Natriumhydrogensulfat als Säure einsetzt.

8. Verfahren nach einem der Ansprüche 1 bis 7, **dadurch gekennzeichnet, dass** man Schritt a) in Gegenwart eines Ruthenium auf einem Kohlenstoffträger umfassenden Katalysators durchführt.

9. Verfahren nach einem der Ansprüche 1 bis 8, **dadurch gekennzeichnet, dass** man Schritt a) in Gegenwart eines mit Eisen dotierten Ruthenium auf einem Träger umfassenden Katalysators durchführt.

10. Verfahren nach einem der Ansprüche 1 bis 9, **dadurch gekennzeichnet, dass** man Schritt a) in Gegenwart eines Katalysators durchführt, der, jeweils bezogen auf den fertigen Katalysator, 0,1 bis 10 Gew.-% Ruthenium und 0,1 bis 5 Gew.-% Eisen auf einem Kohlenstoffträger enthält.

11. Verfahren nach einem der Ansprüche 1 bis 10, **dadurch gekennzeichnet, dass** man in Schritt c) als stark sauren Kationenaustauscher Lewatit® SP112 und/oder Amberlyst™ 15 einsetzt.

12. Verfahren nach einem der Ansprüche 1 bis 11, **dadurch gekennzeichnet, dass** man den Kationenaustauscher in Form eines Festbetts einsetzt.

13. Verfahren nach einem der Ansprüche 1 bis 12, **dadurch gekennzeichnet, dass** man Verfahrensschritt c) kontinuierlich durchführt.

14. Verfahren nach einem der Ansprüche 1 bis 13, **dadurch gekennzeichnet, dass** man Verfahrensschritt c) so durchführt, dass man das zu isomerisierende Gemisch der Verbindungen der Formeln (I) und (III) mehrmals hintereinander mit dem stark sauren Kationentauscher oder auch mit verschiedenen stark sauren Kationentauschern in Kontakt bringt.

## Claims

1. A process for the preparation of cis-2-(2-methylprop-1-enyl)-4-methyltetrahydropyran of the formula (I) 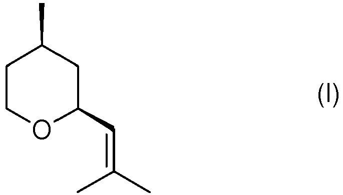 comprising the steps
a) catalytic hydrogenation of 2-(2-methylprop-1-enyl)-4-methylenetetrahydropyran of the formula (II) 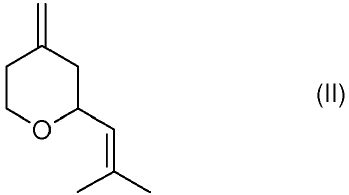 in the presence of hydrogen and a heterogeneous catalyst comprising ruthenium on a support to give a reaction mixture comprising the compound of the formula (I) and trans-2-(2-methylprop-1-enyl)-4-methyltetrahydropyran of the formula (III) 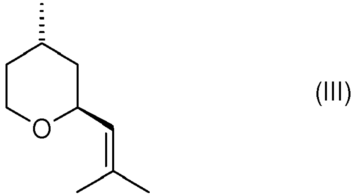 and
b) optionally separating off the compounds of the formula (I) and (III) from the reaction mixture obtained according to step a) and
c) bringing the compounds of the formulae (I) and (III) obtained in step a) or b) into contact with a strongly acidic cation exchanger with isomerization of the compound of the formula (III) to give the compound of the formula (I).

2. The process according to claim 1, additionally comprising the provision of the compound of the formula (II) by reacting 3-methylbut-3-en-1-ol with 3-methylbut-2-en-1-al with the release of water in the presence of an acid and a solvent which forms an azeotrope with water.

3. The process according to claim 2, wherein the water released during the reaction of 3-methylbut-3-en-1-ol with 3-methylbut-2-en-1-al is separated from the reaction mixture by azeotropic distillation with the solvent used.

4. The process according to claim 2 or 3, wherein a solvent which forms an azeotrope with water, selected from the group of solvents comprising toluene, chloroform, n-heptane, cyclohexane, and methylcyclohexane, is used.

5. The process according to any one of claims 2 to 4, wherein the reaction of 3-methylbut-3-en-1-ol with 3-methylbut-2-en-1-al is carried out in the presence of p-toluenesulfonic acid, trifluoroacetic acid or an alkali metal hydrogensulfate.

6. The process according to any one of claims 2 to 5, wherein an alkali metal hydrogensulfate is used as acid.

7. The process according to any one of claims 2 to 6, wherein sodium hydrogensulfate is used as acid.

8. The process according to any one of claims 1 to 7, wherein step a) is carried out in the presence of a catalyst comprising ruthenium on a carbon support.

9. The process according to any one of claims 1 to 8, wherein step a) is carried out in the presence of a catalyst comprising ruthenium doped with iron on a support.

10. The process according to any one of claims 1 to 9, wherein step a) is carried out in the presence of a catalyst which, in each case based on the finished catalyst, comprises 0.1 to 10% by weight of ruthenium and 0.1 to 5% by weight of iron on a carbon support.

11. The process according to any one of claims 1 to 10, wherein, in step c), the strongly acidic cation exchanger used is Lewatit® SP112 and/or Amberlyst™ 15.

12. The process according to any one of claims 1 to 11, wherein the cation exchanger is used in the form of a fixed bed.

13. The process according to any one of claims 1 to 12, wherein process step c) is carried out continuously.

14. The process according to any one of claims 1 to 13, wherein process step c) is carried out such that the mixture of the compounds of the formulae (I) and (III) to be isomerized is brought into contact several times in succession with the strongly acidic cation exchanger or else with different strongly acidic cation exchangers.

## Revendications

1. Procédé pour la préparation de cis-2-(2-méthylprop-1-én-yl)-4-méthyltétrahydropyrane de formule (I) 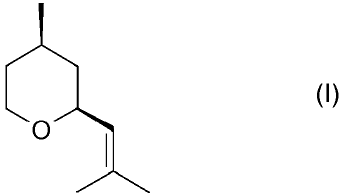 comprenant les étapes de
a) hydrogénation catalytique de 2-(2-méthylprop-1-ényl)-4-méthylènetétrahydropyrane de formule (II) 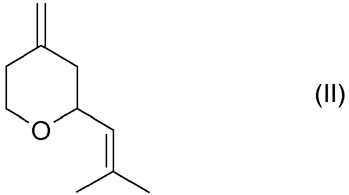 en présence d'hydrogène et d'un catalyseur hétérogène, comprenant du ruthénium sur un support, avec obtention d'un mélange réactionnel comprenant le composé de formule (I) et du trans-2-(2-méthylprop-1-ényl)-4-méthyltétrahydropyrane de formule (III) 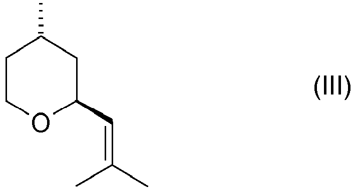 et
b) le cas échéant séparation des composés de formule (I) et (III) du mélange réactionnel obtenu selon l'étape a) et
c) mise en contact des composés des formules (I) et (III) obtenus dans l'étape a) ou b) avec un échangeur cationique fortement acide avec isomérisation du composé de formule (III) en composé de formule (I).

2. Procédé selon la revendication 1, comprenant en outre la mise à disposition du composé de formule (II) par transformation de 3-méthylbut-3-én-1-ol avec du 3-méthylbut-2-én-1-al avec libération d'eau en présence d'un acide et d'un solvant formant un azéotrope avec l'eau.

3. Procédé selon la revendication 2, **caractérisé en ce qu'**on sépare l'eau libérée lors de la transformation du 3-méthylbut-3-én-1-ol avec du 3-méthylbut-2-én-1-al du mélange réactionnel par distillation azéotropique avec le solvant utilisé.

4. Procédé selon la revendication 2 ou 3, **caractérisé en ce qu'**on utilise un solvant formant un azéotrope avec l'eau, choisi dans le groupe des solvants comprenant le toluène, le chloroforme, le n-heptane, le cyclohexane et le méthylcyclohexane.

5. Procédé selon l'une quelconque des revendications 2 à 4, **caractérisé en ce qu'**on réalise la transformation du 3-méthylbut-3-én-1-ol avec du 3-méthylbut-2-én-1-al en présence d'acide p-toluènesulfonique, d'acide trifluoroacétique ou d'un hydrogénosulfate de métal alcalin.

6. Procédé selon l'une quelconque des revendications 2 à 5, **caractérisé en ce qu'**on utilise un hydrogénosulfate de métal alcalin comme acide.

7. Procédé selon l'une quelconque des revendications 2 à 6, **caractérisé en ce qu'**on utilise de l'hydrogénosulfate de sodium comme acide.

8. Procédé selon l'une quelconque des revendications 1 à 7, **caractérisé en ce qu'**on réalise l'étape a) en présence d'un catalyseur comprenant du ruthénium sur un support carboné.

9. Procédé selon l'une quelconque des revendications 1 à 8, **caractérisé en ce qu'**on réalise l'étape a) en présence d'un catalyseur comprenant du ruthénium dopé par du fer sur un support.

10. Procédé selon l'une quelconque des revendications 1 à 9, **caractérisé en ce qu'**on réalise l'étape a) en présence d'un catalyseur qui contient, à chaque fois par rapport au catalyseur fini, 0,1 à 10% en poids de ruthénium et 0,1 à 5% en poids de fer sur un support carboné.

11. Procédé selon l'une quelconque des revendications 1 à 10, **caractérisé en ce qu'**on utilise, dans l'étape c), comme échangeur cationique fortement acide, du Lewatit® SP112 et/ou de l'Amberlyst™ 15.

12. Procédé selon l'une quelconque des revendications 1 à 11, **caractérisé en ce qu'**on utilise l'échangeur cationique sous forme d'un lit fixe.

13. Procédé selon l'une quelconque des revendications 1 à 12, **caractérisé en ce qu'**on réalise l'étape de procédé c) en continu.

14. Procédé selon l'une quelconque des revendications 1 à 13, **caractérisé en ce qu'**on réalise l'étape de procédé c) de manière telle qu'on met en contact le mélange à isomériser des composés des formules (I) et (III) plusieurs fois successivement avec l'échangeur cationique fortement acide ou également avec des échangeurs cationiques fortement acides différents.
